# EUROPÄISCHE PATENTSCHRIFT

(11) **EP 0 861 675 B1**
(45) Veröffentlichungstag und Bekanntmachung des Hinweises auf die Patenterteilung: **12.02.2003**
(21) Anmeldenummer: 98103177.6
(22) Anmeldetag: 24.02.1998
(51) Int. Cl.: A61M 39/16

(54) **Anschlussvorrichtung für medizintechnische Einrichtungen**
Connection device for medical appliances
Dispositif de raccordement pour appareils médicaux

(30) Priorität: 27.02.1997 DE 19707804
(43) Veröffentlichungstag der Anmeldung: 02.09.1998
(73) Patentinhaber: Fresenius Medical Care Deutschland GmbH, 61352 Bad Homburg v.d.H. (DE)
(72) Erfinder: Mathis, Jean-Francois, 49260 St-Cyr en Bourg (FR); Vincent, Eric, 49000 Angers (FR)
(74) Vertreter: Luderschmidt, Schüler & Partner GbR

(56) Entgegenhaltungen:
- WO-A-96/05883
- GB-A- 2 018 927

## Beschreibung

Die Erfindung betrifft eine Anschlußvorrichtung für medizintechnische Einrichtungen.

Zum Anschluß von externen Komponenten an medizintechnische Einrichtungen sind eine Vielzahl von Konnektoren bekannt. Der Zugang zu den medizintechnischen Einrichtungen erfolgt im allgemeinen mittels Steckern, die in passende Buchsen eingeführt werden. An diese Verbindungen sind in der Medizintechnik sowohl auf die Funktionssicherheit als auch auf die Keimfreiheit hohe Anforderungen zu stellen.

Die WO 96/05883 beschreibt eine Anschlußvorrichtung zur Herstellung einer sterilen Verbindung zwischen zwei flüssigkeitsführenden Schlauchleitungssystemen. Die bekannte Vorrichtung weist einen hohlzylindrischen Gehäusekörper auf, der eine Steckbuchse mit einer zylindrischen Ausnehmung aufnimmt. Zum Verschließen der Ausnehmung ist in dem Gehäuse ein zylindrischer Verschlußkörper vorgesehen, der an der Stirnseite eines in dem Gehäusekörper längsverschiebbar geführten Kolbens befestigt ist. An der zylindrischen Ausnehmung sind ein Einlaßkanal zum Zuführen eines flüssigen Desinfektionsmittels und ein Auslaßkanal angeschlossen. Die Anschlußvorrichtung umfaßt darüber hinaus einen Stecker, der zur Herstellung einer Fluidverbindung durch eine seitliche Öffnung in den Gehäusekörper eingeführt und in die Ausnehmung der Steckbuchse passend eingesteckt werden kann.

Zum Desinfizieren wird die Ausnehmung bei abgenommenem Stecker mittels des Verschlußkörpers verschlossen, woraufhin ein Desinfektionsmittel über den Einlaßkanal in die Kammer geleitet und wieder abgeführt wird.

Die bekannte Anschlußvorrichtung erlaubt eine Desinfektion der flüssigkeitsführenden Bereiche. Nachteilig ist jedoch, daß der Anschluß nur schwer zugänglich ist, was die Handhabung erschwert.

Der Erfindung liegt die Aufgabe zugrunde, eine einfach zu handhabende Anschlußvorrichtung für medizintechnische Einrichtungen zu schaffen, die sich nach jeder Konnektierung wirkungsvoll desinfizieren läßt.

Die Lösung dieser Aufgabe erfolgt mit den im Patentanspruch 1 angegebenen Merkmalen.

Die erfindungsgemäße Anschlußvorrichtung weist einen in der Ausnehmung des Gehäusekörpers verschiebbaren Konnektor auf, der mit dem Verschlußkörper derart gekoppelt ist, daß er bei verschlossener Ausnehmung zurückgezogen und bei geöffneter Ausnehmung nach außen vorgeschoben und somit leicht zugänglich ist. Zum Einleiten des Desinfektions- oder Spülvorganges braucht die Ausnehmung lediglich mittels des Verschlußkörper verschlossen zu werden, wodurch sich der Konnektor selbsttätig zurückzieht. In der verschlossenen Kammer kann der Konnektor dann mit einer Desinfektions- oder Spülflüssigkeit wirkungsvoll umspült werden. Die Zuführung der Desinfektions- oder Spülflüssigkeit kann automatisch dann in Gang gesetzt werden, wenn der Verschlußkörper die Ausnehmung verschließt.

Die erfindungsgemäße Anschlußvorrichtung kann insbesondere bei medizintechnischen Apparaten, z.B. Dialysemaschinen, zum Anschluß von Leitungen Verwendung finden, die Blut, Dialyse- oder Substituatflüssigkeit führen. Die Anschlußvorrichtung ist aber auch in allen anderen Anwendungsfällen einsetzbar, in denen ein leicht zugänglicher Anschluß geschaffen werden soll, der desinfizier- oder spülbar ist.

Der Konnektor ist vorteilhafterweise in der vorgeschobenen bzw, zurückgezogenen Position durch Federkraft festgelegt, was die Handhabung weiter vereinfacht. Die Federkraft sollte so bemessen sein, daß der Verschlußkörper noch ohne großen Kraftaufwand verstellbar ist.

Vorzugsweise ist der Verschlußkörper als Klappdeckel ausgebildet. Der Verschlußkörper kann aber auch ein verschiebbarer Deckel sein.

Der Konnektor kann je nach Anwendungsfall unterschiedlich ausgebildet sein, um an die bekannten Konnektoren zum Verbinden von Schlauchleitungen, z.B. Luer-Kupplungen oder Hansen-Kupplungen, angeschlossen werden zu können. Zum Anschluß von flüssigkeitsführenden Schlauchleitungen, die selbst über kein komplementäres Anschlußstück verfügen, weist der Konnektor an seinem freien Ende vorteilhafterweise einen rohrförmigen Abschnitt auf, der ein leichtes Aufschieben der Leitung ermöglicht.

In einer bevorzugten Aussführungsform ist der Konnektor über ein Gelenkgetriebe mit dem Verschlußkörper gekoppelt, das bei einem relativ geringen technischen Aufwand funktionssicher arbeitet. Es ist aber auch eine elektromagnetische Betätigung des Verschlußkörpers und des Konnektors möglich.

Weitere vorteilhafte Ausführungsformen der Erfindung sind Gegenstand der Unteransprüche.

Im folgenden wird ein Ausführungsbeispiel der Erfindung unter Bezugnahme auf die Zeichnungen näher erläutert.

Es zeigen:
- Fig. 1: die Anschlußvorrichtung in geschnittener Darstellung, wobei der Klappdeckel geöffnet und der Konnektor vorgeschoben ist,
- Fig. 2: eine perspektivische Darstellung der Anschlußvorrichtung in der Vorderansicht, wobei der Konnektor vorgeschoben ist,
- Fig. 3: eine perspektivische Darstellung der Anschlußvorrichtung in der Rückansicht, wobei der Konnektor vorgeschoben ist,
- Fig. 4: die Anschlußvorrichtung in geschnittener Darstellung, wobei der Klappdeckel geschlossen und der Konnektor zurückgezogen ist,
- Fig. 5: eine perspektivische Darstellung der Anschlußvorrichtung in der Vorderansicht, wobei der Konnektor zurückgezogen ist und
- Fig. 6: eine perspektivische Darstellung der Anschlußvorrichtung in der Rückansicht, wobei der Konnektor zurückgezogen ist.

Die Anschlußvorrichtung weist eine Frontplatte 1 mit einem kreisförmigen Ausschnitt 2 auf. An der Rückseite der Frontplatte 1, bei der es sich auch um die Seitenwand des Gehäuses eines medizintechnischen Apparates, z.B. eine Hämodialysemaschine, handeln kann, befindet sich hinter dem kreisförmigen Ausschnitt 2 ein hohlzylindrischer Körper 3, in dem ein Konnektor 4 angeordnet ist, der zum Anschluß einer nicht dargestellten Schlauchleitung für die Versorgung einer externen Komponente mit einer Flüssigkeit, z.B. Dialysier- oder Substituatflüssigkeit, dient, die von dem medizinischen Apparat bereitgestellt wird.

Der Konnektor 4 weist einen rohrförmigen vorderen Abschnitt 5 zum Aufschieben der Schlauchleitung auf, an den sich ein rohrförmiger hinterer Abschnitt 6 anschließt, der einen größeren Durchmesser als der vordere Abschnitt 5 hat. Der hintere Abschnitt 6 des Konnektors 4 geht in ein kurzes rohrförmiges Anschlußstück 7 zum Anschluß der Schlauchleitung über, die zu dem Aggregat, z.B. der Dialysierflüssigkeits- oder Substituatpumpe, des medizintechnischen Apparates führt, das die Flüssigkeit bereitstellt.

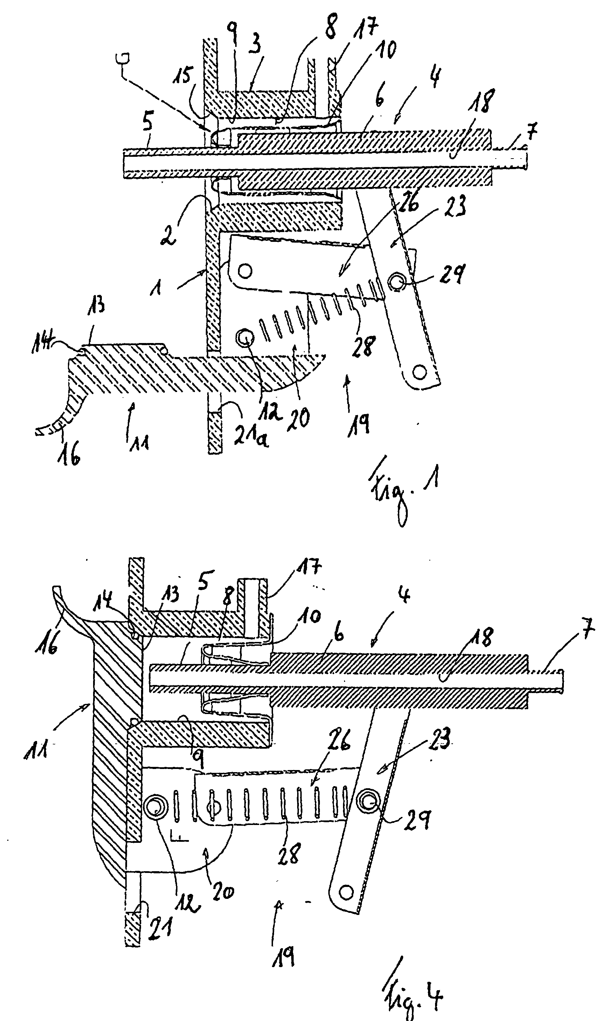

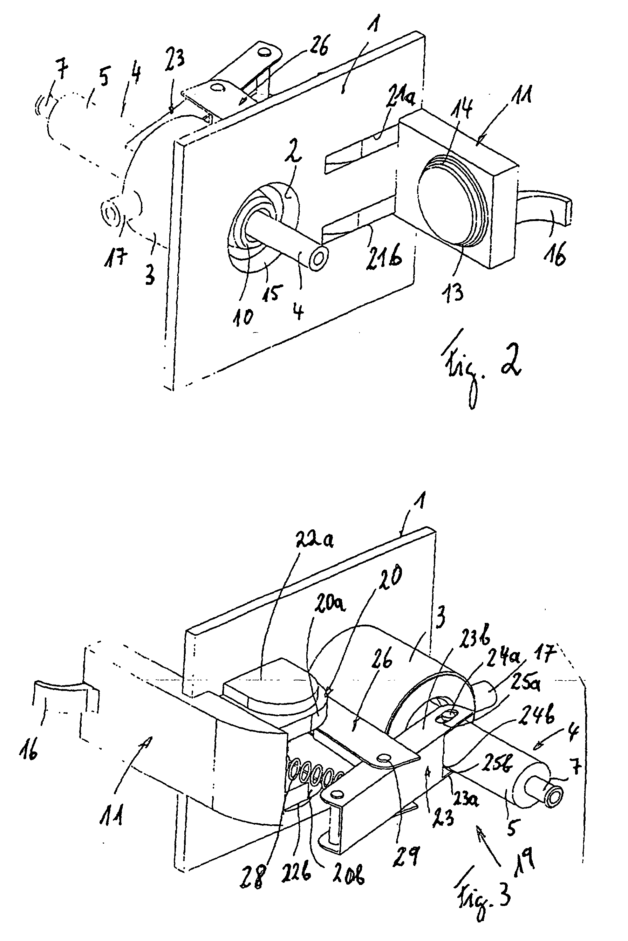

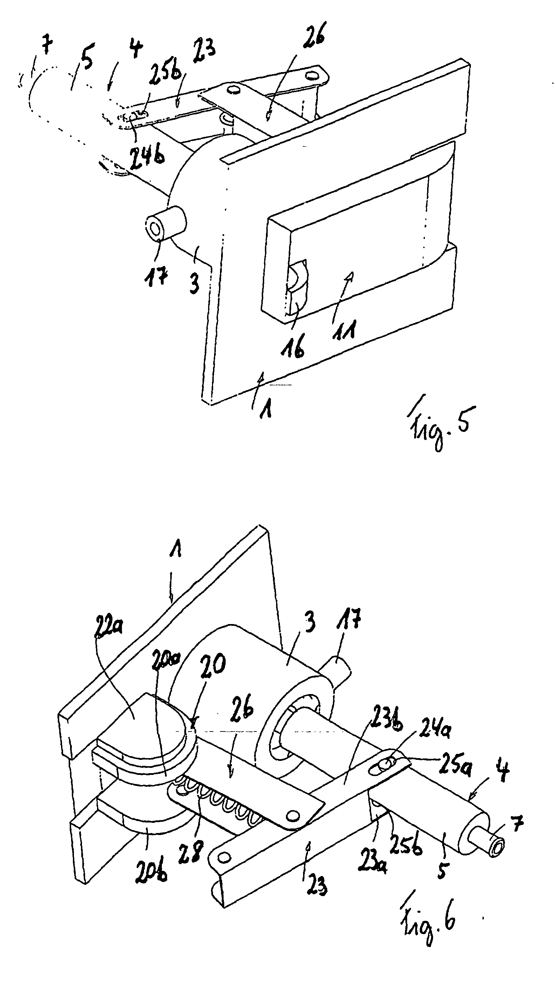

Der Konnektor 4 ist in dem hohlzylindrischen Körper 3 in Längsrichtung verschiebbar geführt, wobei dieser zwei Stellungen einnehmen kann. In der vorgeschobenen Stellung (Fign. 1 bis 3) erstreckt sich der rohrförmige vordere Abschnitt 5 des Konnektors 4 durch die kreisförmige Ausnehmung 2 der Frontplatte 1 nach außen, während der vordere Abschnitt des Konnektors in der zurückgezogenen Position (Fign. 4 bis 6) unter Bildung eines Ringspaltes 8 in der Ausnehmung 9 des Gehäusekörpers versenkt ist. In dieser Stellung befindet sich der hintere rohrförmige Abschnitt 6 des Konnektors 4 außerhalb der Ausnehmung 9. Der Konnektor 4 ist gegenüber dem hohlzylindrischen Körper 3 durch eine flexible Membran 10 abgedichtet, deren innerer Rand an dem hinteren Ende des vorderen Abschnitts 5 des Konnektors 4 und dessen äußerer Rand an dem hohlzylindrischen Körper 3 befestigt ist.

In der zurückgezogenen Position des Konnektors 4 ist die kreisförmige Ausnehmung 2 in der Frontplatte 1 von einem Klappdeckel 11 verschlossen, der an der Frontplatte um eine quer zur Längsachse des Konnektors verlaufende Achse schwenkbar gelagert ist. Der in seiner geschlossenen Stellung flach auf der Frontplatte aufliegende Klappdeckel 11 weist einen Ansatz 13 mit einem Dichtring 14 auf, der den Deckel gegenüber der Schrägfläche 15 der Frontplatte abdichtet. Um den Klappdeckel besser greifen zu können, ist dieser mit einem Griffteil 16 versehen.

An der Wand des hohlzylindrischen Körpers 3 ist ein Auslaßkanal 17 für eine Desinfektions- oder Spülflüssigkeit vorgesehen, die bei geschlossenem Klappdeckel 11 über den Kanal 18 des Konnektors in die als Kammer geschaltete Ausnehmung geleitet werden kann.

Der Klappdeckel 11 ist mit dem Konnektor 4 über ein Gelenkgetriebe 19 derart gekoppelt, daß der Konnektor beim Öffnen des Deckels aus seiner zurückgezogenen Stellung zum Anschluß der Schlauchleitung automatisch nach außen vorgeschoben wird. Das Gelenkgetriebe 19 wird nachfolgend im einzelnen beschrieben.

An der Rückseite des Klappdeckels 11 ist ein Getriebeglied 20 befestigt, das aus zwei parallelen Platten 20a, 20b besteht, die durch zwei parallele Schlitze 21a, 21b in der Frontplatte nach hinten vorstehen. Die Platten 20a, 20b sind auf einer Achse 12 drehbar gelagert, die sich zwischen den an der Rückseite der Frontplatte befestigten Seitenwänden 22a, 22b erstreckt.

Ein zweites Getriebeglied 23 ist an dem hinteren Abschnitt des Konnektors 4 um eine zur Schwenkachse des Klappdeckels 11 parallele Achse schwenkbar angelenkt. Das zweite Getriebeglied 23 ist ein U-förmiges Profilstück, dessen Seitenteile 23a, 23b an einem Ende vorstehen und an dem hinteren Abschnitt 5 des Konnektors 4 anliegen. Die Seitenteile 23a, 23b sind an seitlichen Zapfen 24a, 24b des hinteren Abschnitts des Konnektors 5 befestigt, die in Langlöcher 25a, 25b an den vorstehenden Enden 23a, 23b des zweiten Getriebeglieds 23 greifen.

Darüber hinaus umfaßt das Gelenkgetriebe 19 ein Kopplungsglied 26 aus einem U-förmigen Profilstück, das einerseits an dem ersten Getriebeglied 20 in dessen Randbereich und andererseits an den Seitenteilen 23a, 23b des zweiten Getriebeglieds 23 zwischen dessen Enden angelenkt ist.

Zwischen der Achse 12 des ersten Getriebsgliedes 20 und dem Gelenkzapfen, 29 der das Kopplungsglied 26 mit dem zweiten Getriebeglied 23 verbindet, ist eine Zugfeder 28 gespannt, die den Klappdeckel 11 festhält.

Da der Konnektor 4 vor dem Öffnen des Klappdeckels 11 hermetisch abgeschlossen ist, wird eine Kontamination wirksam vermieden. Beim Öffnen des Klappdeckels entgegen der Federkraft der Zugfeder 28 wird das erste Getriebeglied 20 verschwenkt, wodurch der Konnektor 4 über das Kopplungsglied 26 und das zweite Getriebeglied 23 vorgeschoben wird. In dieser Stellung ist der vordere Abschnitt 5 des Konnektors 4 leicht zugänglich, so daß die Schlauchleitung leicht aufgeschoben oder aufgedreht werden kann. Beim Schließen des Klappdeckels 11 entgegen der Federkraft der Zugfeder 28 wird der Konnektor 4 wieder in die Kammer 9 des Gehäusekörpers zurückgeschoben.

Wird in der zurückgezogenen Position nun ein Desinfektions- oder Spülmittel in den Konnektor 4 geleitet, so wird der vordere Abschnitt 5 des Konnektors 4 von der Flüssigkeit in der Kammer 9 vollständig umspült. Die verbrauchte Flüssigkeit kann über den Auslaßkanal 17 abgeleitet werden. Es ist aber auch möglich, die Flüssigkeit in einem geschlossenen Spülkreislauf wieder über den Konnektor 4 der Kammer 9 zuzuführen.

Die Anschlußvorrichtung kann z.B. für ein Hämodialysegerät, Hämofiltrations- und Hämodiafiltrationsgerät zum Anschluß eines extrakorporalen Blutschlauchsystems sowie für ein Gerät zur Wiederaufbereitung von Disposables Verwendung finden. Mit der Vorrichtung kann auch on-line aufbereitete Substituatlösung dem Blutschlauchsystem zugeführt werden. Mit der Anschlußvorrichtung kann auch eine Reinigungsflüssigkeit, beispielsweise für Disposables zur Verfügung gestellt werden. Darüber hinaus kann die Anschlußvorrichtung zum Befüllen von Disposables mit medizinischen Flüssigkeiten auch im Rahmen von produktionstechnischen Anlagen Verwendung finden.

## Patentansprüche

1. Anschlußvorrichtung für medizintechnische Einrichtungen mit
einem Gehäusekörper (1, 3), in dem eine zur Umgebung offene Ausnehmung (9) ausgebildet ist,
einem in der Ausnehmung angeordneten Konnektor (4) zur Flüssigkeitsdurchleitung, der durch die Öffnung der Ausnehmung (9) im Gehäusekörper von außen zugänglich ist,
einem Verschlußkörper (11), der zwischen einer die Ausnehmung von außen verschließenden Stellung und einer die Ausnehmung öffnenden Stellung verstellbar ist, und
einem an der Ausnehmung (9) angeschlossenen Auslaßkanal (17),
**dadurch gekennzeichnet,**
**daß** der Konnektor (4) in der Ausnehmung (9) verschiebbar ist, wobei der Konnektor (4) mit dem Verschlußkörper (11) derart gekoppelt ist, daß der Konnektor bei verschlossener Ausnehmung in die Ausnehmung zurückgezogen und bei geöffneter Ausnehmung aus der Ausnehmung heraus vorgeschoben ist.

2. Anschlußvorrichtung nach Anspruch 1, **dadurch gekennzeichnet, daß** der Konnektor (4) in der vorgeschobenen und zurückgezogenen Position durch Federkraft festgelegt ist.

3. Anschlußvorrichtung nach Anspruch 1 oder 2, **dadurch gekennzeichnet, daß** der Verschlußkörper als Klappdeckel (11) ausgebildet ist.

4. Anschlußvorrichtung nach einem der Ansprüche 1 bis 3, **dadurch gekennzeichnet, daß** die Ausnehmung von einem hohlzylindrischen Teil (3) des Gehäusekörpers gebildet wird, daß der Konnektor (4) an seinem freien Ende einen rohrförmigen Abschnitt (5), vorzugsweise mit einem Anschlußstück, aufweist, der unter Bildung eines Ringspaltes (8) in die Ausnehmung eintaucht, wobei der rohrförmige Abschnitt (5) des Konnektors (4) gegenüber dem hohlzylindrischen Teil (3) des Gehäusekörpers (1, 3) abgedichtet ist.

5. Anschlußvorrichtung nach einem der Ansprüche 1 bis 4, **dadurch gekennzeichnet, daß** der Konnektor (4) über ein Gelenkgetriebe (19) mit dem Verschlußkörper (11) gekoppelt ist.

6. Anschlußvorrichtung nach Anspruch 5, **dadurch gekennzeichnet, daß** der Verschlußkörper (11) um eine quer zur Längsachse des Konnektors verlaufende Achse (12) schwenkbar an dem Gehäusekörper (1, 3) gelagert ist und daß das Gelenkgetriebe (19) ein mit dem Verschlußkörper starr verbundenes Getriebeglied (20), ein an dem Konnektor angelenktes Getriebeglied (23), das um eine zu der Schwenkachse des Verschlußkörpers parallele Achse schwenkbar an dem Konnektor (4) angelenkt ist, und ein die beiden Getriebeglieder (20, 23) verbindendes Kopplungsglied (26) aufweist.

7. Anschlußvorrichtung nach Anspruch 6, **dadurch gekennzeichnet, daß** zwischen dem Kopplungsglied (26) und dem Gehäusekörper (1, 3) eine Zugfeder (28) gespannt ist.

8. Anschlußvorrichtung nach Anspruch 7, **dadurch gekennzeichnet, daß** das konnektorseitige Drehgelenk in Langlöchern (25a, 25b) geführte Gelenkzapfen (24a, 24b) aufweist.

9. Anschlußvorrichtung nach einem der Ansprüche 4 bis 8, **dadurch gekennzeichnet, daß** eine den Konnektor (4) gegenüber dem hohlzylindrischen Teil (3) des Gehäusekörpers (1, 3) abdichtende flexible Membran (10) vorgesehen ist.

## Claims

1. Connecting device for technical medical equipment with a housing body (1, 3) in which a recess (9) open to the environment is formed,
a connector (4) for the liquid connecting line, which is accessible from the outside through the opening of the recess (9) in the housing body,
a sealing body (11) that can be adjusted between a position that closes the recess from outside and a position which opens the recess, and
a drain channel (17) connected to the recess (9),
**characterised in that**,
the connector (4) can slide in the recess (9), whereby the connector (4) is connected to the sealing body (11) in such a manner that the connector is withdrawn into the recess with the recess closed and is extended outwards from the recess with the recess open.

2. Connecting device in accordance with claim 1, **characterised in that** the connector (4) is spring loaded in the extended and retracted positions.

3. Connecting device in accordance with claim 1 or 2, **characterised in that** the sealing body is designed as a hinged cover (11).

4. Connecting device in accordance with one of claims 1 to 3, **characterised in that** the recess is formed by a hollow cylindrical part (3) of the housing body, that the connector (4) has a tubular section (5) at its free end, preferably with a connecting piece that dips into the recess forming an annular gap (8), whereby the tubular section (5) of the connector (4) is sealed against the hollow cylindrical part (3) of the housing body (1, 3).

5. Connecting device in accordance with one of claims 1 to 4, **characterised in that** the connector (4) is coupled by means of a linkage mechanism (19) to the sealing body (11).

6. Connecting device in accordance with claim 5, **characterised in that** the sealing body (11) is mounted on the housing body (1, 3) in such a way that it can swivel about an axis (12) running transverse to the longitudinal axis of the connector and that the linkage mechanism (19) consists of a linkage element (20) rigidly connected to the sealing body, a linkage element (23) hinge-connected to the connector (4) in such a way that it can swivel about an axis parallel to the swivelling axis of the sealing body, and a coupling element (26) connecting both linkage elements (20, 23).

7. Connecting device in accordance with claim 6, **characterised in that** a tension spring (28) is held under tension between the coupling element (26) and the housing body (1, 3).

8. Connecting device in accordance with claim 7, **characterised in that** the connector end of the rotating link has link spigots (24a, 24b) guided in slots (25a, 25b).

9. Connecting device in accordance with one of claims 4 to 8, **characterised in that** a flexible diaphragm (10) is provided that seals the connector (4) against the hollow cylindrical part (3) of the housing body (1, 3).

## Revendications

1. Dispositif de raccordement pour appareils médicaux avec un corps de boîtier (1, 3), dans lequel est conçue une excavation ouverte (9) à l'environnement, un connecteur (4) placé dans l'excavation pour la conduite du liquide et qui est accessible de l'extérieur par l'ouverture de l'excavation (9) dans le corps de boîtier, un corps de verrouillage (11), qui peut être réglé entre une position fermant l'excavation de l'extérieur et une position ouvrant l'excavation, et un canal d'évacuation (17) raccordé à l'excavation (9), **caractérisé en ce que** le connecteur (4) peut être poussé dans l'excavation (9), moyennant quoi le connecteur (4) est couplé au corps de verrouillage (11) de telle sorte que le connecteur se rétracte dans l'excavation lorsque celle-ci est fermée et se pousse vers l'avant hors de l'excavation lorsque celle-ci est ouverte.

2. Dispositif de raccordement selon la revendication 1, **caractérisé en ce que** le connecteur (4) est maintenu en position poussée et rétractée par la tension d'un ressort.

3. Dispositif de raccordement selon la revendication 1 ou 2, **caractérisé en ce que** le corps de verrouillage est conçu comme couvercle rabattable (11).

4. Dispositif de raccordement selon l'une des revendications 1 à 3, **caractérisé en ce que** l'excavation est formée par une partie creuse cylindrique (3) du corps de boîtier, que le connecteur (4) présente à son extrémité libre un segment tubulaire (5), de préférence avec pièce de raccordement, segment qui plonge dans l'excavation par la formation d'un interstice annulaire (8), moyennant quoi le segment tubulaire (5) du connecteur (4) est étanche par rapport à la partie creuse cylindrique (3) du corps de boîtier (1, 3).

5. Dispositif de raccordement selon l'une des revendications 1 à 4, **caractérisé en ce que** le connecteur (4) est couplé au corps de verrouillage (11) par un mécanisme articulé (19).

6. Dispositif de raccordement selon la revendication 5, **caractérisé en ce que** le corps de verrouillage (11) est monté sur le corps de boîtier (1, 3) de façon à pivoter autour d'un axe (12) passant à l'oblique de l'axe longitudinal du connecteur et **en ce que** le mécanisme articulé (19) présente un élément de mécanisme (20) relié de façon rigide au corps de verrouillage, un élément de mécanisme (23) qui s'articule sur le connecteur (4) en pivotant autour d'un axe parallèle à l'axe de pivotement du corps de verrouillage, articulé sur le connecteur et un élément de couplage (26) reliant les deux éléments de mécanisme (20, 23).

7. Dispositif de raccordement selon la revendication 6, **caractérisé en ce qu'**un ressort de traction (28) est tendu entre l'élément de couplage (26) et le corps de boîtier (1, 3).

8. Dispositif de raccordement selon la revendication 7, **caractérisé en ce que** l'articulation rotative située côté connecteur présente des chevilles d'articulation (24a, 24b) s'insérant dans des trous ovales (25a, 25b).

9. Dispositif de raccordement selon l'une des revendications 4 à 8, **caractérisé en ce qu'**est prévue une membrane flexible (10) assurant l'étanchéité du connecteur (4) face à la partie creuse cylindrique (3) du corps de boîtier (1, 3).
